# EUROPÄISCHE PATENTSCHRIFT

(11) **EP 1 455 785 B1**
(45) Veröffentlichungstag und Bekanntmachung des Hinweises auf die Patenterteilung: **04.03.2009**
(21) Anmeldenummer: 02791733.5
(22) Anmeldetag: 28.11.2002
(51) Int. Cl.: A61K 31/4418, C07D 213/85, C07D 417/12, C07D 417/14, A61P 25/00, A61P 9/00, A61P 29/00, A61P 11/06

(54) **SUBSTITUIERTE 2-THIO-3,5-DICYANO-4-PHENYL-6-AMINOPYRIDINE UND IHRE VERWENDUNG**
SUBSTITUTED 2-THIO-3,5-DICYANO-4-PHENYL-6-AMINOPYRIDINES AND THE USE OF THE SAME
2-THIO-3,5-DICYANO-4-PHENYL-6-AMINOPYRIDINE SUBSTITUEES ET LEUR UTILISATION

(30) Priorität: 11.12.2001 DE 10160661; 21.08.2002 DE 10238113
(43) Veröffentlichungstag der Anmeldung: 15.09.2004
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); SHIMADA, Mitsuyuki, Higashigawa-cho, Nara 630-8323 (JP); HÜBSCH, Walter, 42113 Wuppertal (DE); DIEDRICHS, Nicole, 42103 Wuppertal (DE); KRAHN, Thomas, 58135 Hagen (DE); HENNINGER, Kerstin, 42115 Wuppertal (DE); STASCH, Johannes-Peter, 42651 Solingen (DE); WISCHNAT, Ralf, 51061 Köln (DE)
(86) Internationale Anmeldenummer: PCT/EP2002/013432
(87) Internationale Veröffentlichungsnummer: WO 2003/053441

(56) Entgegenhaltungen:
- WO-A-01/25210
- WO-A-02/070485
- WO-A-02/079195
- WO-A-03/008384

## Beschreibung

Die vorliegende Erfindung betrifft substituierte 2-Thio-3,5-dicyano-4-phenyl-6-aminopyridine, ein Verfahren zu ihrer Herstellung und ihre Verwendung als Arzneimittel.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehirn).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt; sind bisher die Subtypen A1, A2a, A2b und A3. Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Als "selektiv" werden im Rahmen der vorliegenden Erfindung solche Adenosin-Rezeptor-Liganden bezeichnet, bei denen einerseits eine deutliche Wirkung an einem oder mehreren Adenosin-Rezeptor-Subtypen und andererseits keine oder eine deutliche schwächere Wirkung (Faktor 10 oder geringer) an einem oder mehreren anderen Adenosin-Rezeptor-Subtypen zu beobachten ist, wobei bezüglich der Testmethoden für die Wirk-Selektivität Bezug genommen wird auf die im Abschnitt A. II. beschriebenen Testmethoden.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosin-rezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis." in *J. Biol. Chem.* 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells" in *Naunyn Schmiedebergs Arch. Pharmacol.* 357 (1998) Seiten 1-9, deren Offenbarung hiermit im vollen Umfang durch Bezugnahme eingeschlossen ist).

Im Falle von A1-Agonisten (Kopplung bevorzugt über Gᵢ-Proteine) wird dabei eine Abnahme des intrazellulären cAMP-Gehaltes (bevorzugt nach direkter Vorstimulation der Adenylatzyklase durch Forskolin), im Falle von A1-Antagonisten eine Zunahme des intrazellulären cAMP-Gehaltes beobachtet (bevorzugt nach Vorstimulation mit Adenosin oder Adenosin ähnlichen Substanzen plus direkter Vorstimulation der Adenylatzyklase durch Forskolin). Entsprechend führen A2a und A2b-Agonisten (Kopplung bevorzugt über Gₛ-Proteine) zu einer Zunahme und A2a und A2b-Antagonisten zu einem Abnahme im cAMP-Gehalt der Zellen. Im Falle der A2-Rezeptoren ist eine direkte Vorstimulation der Adenylatzyklase durch Forskolin nicht hilfreich.

Bei den aus dem Stand der Technik bekannten, als "adenosinrezeptor-spezifisch" geltenden Liganden handelt es sich überwiegend um Derivate auf Basis des natürlichen Adenosins (S.-A. Poulsen und R. J. Quinn, "Adenosine receptors: new opportunities for future drugs" in *Bioorganic and Medicinal Chemistry* 6 (1998) Seiten 619 bis 641). Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet.

Darüber hinaus sind aus WO 01/25210 2-Thio-3,5-dicyano-4-aryl-6-aminopyridine bekannt, die den erfindungsgemäßen Verbindungen strukturell ähnlich sind. Die dort beschriebenen Verbindungen haben allerdings weniger vorteilhafte pharmakokinetische Eigenschaften, insbesondere nur eine geringe Bioverfügbarkeit nach oraler Gabe.

Aufgabe der vorliegenden Erfindung ist nunmehr die Auffindung bzw. Bereitstellung von Verbindungen, die die Nachteile des Standes der Technik vermeiden bzw. eine verbesserte Bioverfiigbarkeit besitzen.

Die vorliegende Erfindung betrifft somit Verbindungen der Formel (I) 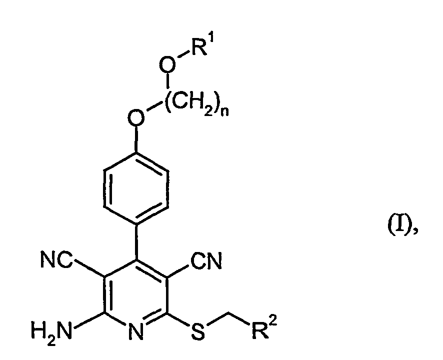 worin
- n: eine Zahl 2, 3 oder 4 bedeutet,
- R¹: Wasserstoff oder (C₁-C₄)-Alkyl bedeutet
und
- R²: Pyridyl oder Thiazolyl bedeutet, das seinerseits durch (C₁-C₄)-Alkyl, Halogen, Amino, Dimethylamino, Acetylamino, Guanidino, Pyridylamino, Thienyl, Furyl, Imidazolyl, Pyridyl, Morpholinyl, Thiomorpholinyl, Piperidinyl, Piperazinyl, N-(C₁-C₄)-Alkylpiperazinyl, Pyrrolidinyl, Oxazolyl, Isoxazolyl, Pyrimidinyl, Pyrazinyl, gegebenenfalls durch (C₁-C₄)-Alkyl substituiertes Thiazolyl oder gegebenenfalls bis zu dreifach durch Halogen, (C₁-C₄)-Alkyl oder (C₁-C₄)-Alkoxy substituiertes Phenyl substituiert sein kann,
und ihre Salze, Hydrate, Hydrate der Salze und Solvate.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die übrigen Tautomeren der Verbindungen der Formel (I) und deren Salze.

Salze der Verbindungen der Formel (I) können physiologisch unbedenkliche Salze der erfindungsgemäßen Stoffe mit Mineralsäuren, Carbonsäuren- oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldüsopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:

Halogen steht im allgemeinen für Fluor, Chlor, Brom oder Iod. Bevorzugt sind Fluor, Chlor oder Brom. Ganz besonders bevorzugt sind Fluor oder Chlor.

(C₁-C₄)-Alkyl steht im allgemeinen für einen geradkettigen oder verzweigten Alkylrest mit 1 bis 4 Kohlenstoffatomen. Beispielsweise seien genannt: Methyl, Ethyl, n-Propyl, Isopropyl, n-Butyl, sec-Butyl, Isobutyl und tert.-Butyl.

(C₁-C₄)-Alkoxy steht im allgemeinen für einen geradkettigen oder verzweigten Alkoxyrest mit 1 bis 4 Kohlenstoffatomen. Beispielsweise seien genannt: Methoxy, Ethoxy, n-Propoxy, Isopropoxy, n-Butoxy, sec-Butoxy, Isobutoxy und tert.-Butoxy.

Bevorzugt sind Verbindungen der Formel (I)
worin
- n: die Zahl 2 bedeutet,
- R¹: Wasserstoff, Methyl oder Ethyl bedeutet
und
- R²: Pyridyl oder Thiazolyl bedeutet, das seinerseits durch Methyl, Ethyl, Fluor, Chlor, Amino, Dimethylamino, Acetylamino, Guanidino, 2-Pyridylamino, 4-Pyridylamino, Thienyl, Pyridyl, Morpholinyl, Piperidinyl, gegebenenfalls durch Methyl substituiertes Thiazolyl oder gegebenenfalls bis zu dreifach durch Chlor oder Methoxy substituiertes Phenyl substituiert sein kann,
und ihre Salze, Hydrate, Hydrate der Salze und Solvate.

Besonders bevorzugt sind Verbindungen der Formel (I), worin R¹ Wasserstoff oder Methyl bedeutet.

Besonders bevorzugt sind ebenfalls Verbindungen der Formel (I), worin
- n: die Zahl 2 bedeutet,
- R¹: Wasserstoff oder Methyl bedeutet
und
- R²: Pyridyl oder Thiazolyl bedeutet, das seinerseits durch Methyl, Chlor, Amino, Dimethylamino, Acetylamino, Guanidino, 2-Pyridylamino, 4-Pyridylamino, Thienyl, Pyridyl, Morpholinyl, 2-Methyl-thiazol-5-yl, Phenyl, 4-Chlorphenyl oder 3,4,5-Trimethoxyphenyl substituiert sein kann,
und ihre Salze, Hydrate, Hydrate der Salze und Solvate.

Ganz besonders bevorzugt ist die Verbindung aus Beispiel 6 mit der folgenden Struktur 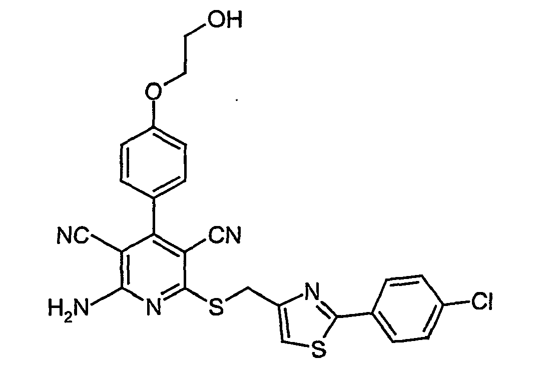 und ihre Salze, Hydrate, Hydrate der Salze und Solvate.

Gegenstand der vorliegenden Erfindung ist auch ein Verfahren zur Herstellung der Verbindungen der Formel (I), dass dadurch gekennzeichnet ist, dass man
Verbindungen der Formel (II) 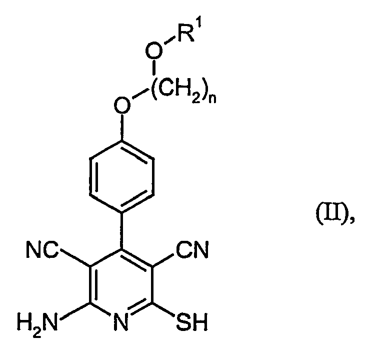 worin
n und R¹ die zuvor angegebene Bedeutung haben,
mit Verbindungen der Formel (III)

R²-CH₂-X (III),

worin
- R²: die zuvor angegebene Bedeutung hat und X für eine geeignete Abgangsgruppe, beispielhaft und vorzugsweise für Halogen, insbesondere Chlor, Brom oder Iod, oder für Mesylat, Tosylat, Triflat oder 1-Imidazolyl, steht,
gegebenenfalls in Anwesenheit einer Base, umsetzt.

Das zuvor beschriebene Verfahren kann durch folgendes Formelschema beispielhaft erläutert werden:

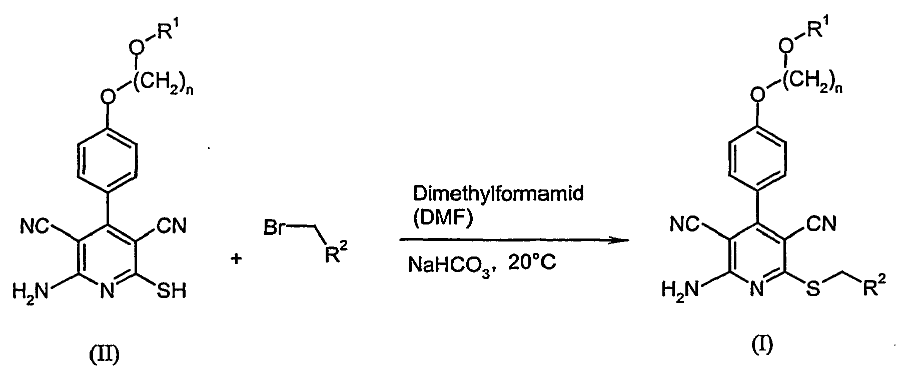

Als Lösemittel für das erfindungsgemäße Verfahren eignen sich alle organischen Lösemittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösemittel ebenso geeignet. Bevorzugt ist Dimethylformamid. Ebenso ist es möglich, Gemische der zuvor genannten Lösemittel einzusetzen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Alkalihydrogencarbonate wie Natrium- oder Kaliumhydrogencarbonat oder Alkalialkoholate wie Natrium- oder Kaliummethanolat, Natrium- oder Kaliumethanolat oder Kalium-tert.-butylat oder Amide wie Natriumamid, Lithium-bis-(trimethylsilyl)amid oder Lithiumdiisopropylamid oder metallorganische Verbindungen wie Butyllithium oder Phenyllithium oder 1,8-Diazabicyclo[5.4.0]undec-7-en (DBU) oder 1,5-Diazabicyclo[4.3.0]non-5-en (DBN) oder aber Amine wie Triethylamin und Pyridin. Bevorzugt sind die Alkalicarbonate und -hydrogencarbonate.

Die Base kann hierbei in einer Menge von 1 bis 10 Mol, bevorzugt von 1 bis 5 Mol, insbesondere 1 bis 4 Mol, bezogen auf 1 Mol der Verbindungen der Formel (II) eingesetzt werden.

Die Reaktion erfolgt im allgemeinen in einem Temperaturbereich von -78°C bis zur +140°C, bevorzugt im Bereich von -78°C bis +40°C, insbesondere bei Raumtemperatur.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Die Verbindungen der Formel (II) sind dem Fachmann an sich bekannt oder nach üblichen, literaturbekannten Methoden herstellbar, beispielsweise durch Umsetzung der entsprechenden Benzaldehyde mit Cyanthioacetamid. Insbesondere kann auf die folgenden Druckschriften verwiesen werden, deren jeweiliger Inhalt durch Bezugnahme eingeschlossen wird:
● Dyachenko et al., Russian Journal of Chemistry, Vol. 33, No. 7, 1997, Seiten 1014 bis 1017 und Vol. 34, No. 4, 1998, Seiten 557 bis 563;
● Dyachenko et al., Chemistry of Heterocyclic Compounds, Vol. 34, No. 2, 1998, Seiten 188 bis 194;
● Qintela et al., European Journal of Medicinal Chemistry, Vol. 33, 1998, Seiten 887 bis 897;
● Kandeel et al., Zeitschrift für Naturforschung 42b, 107 bis 111 (1987).

So können Verbindungen der Formel (II) beispielsweise auch aus Verbindungen der Formel (IV) durch Umsetzung mit einem Alkalisulfid hergestellt werden. Diese Herstellungsmethode kann durch folgendes Formelschema beispielhaft erläutert werden:

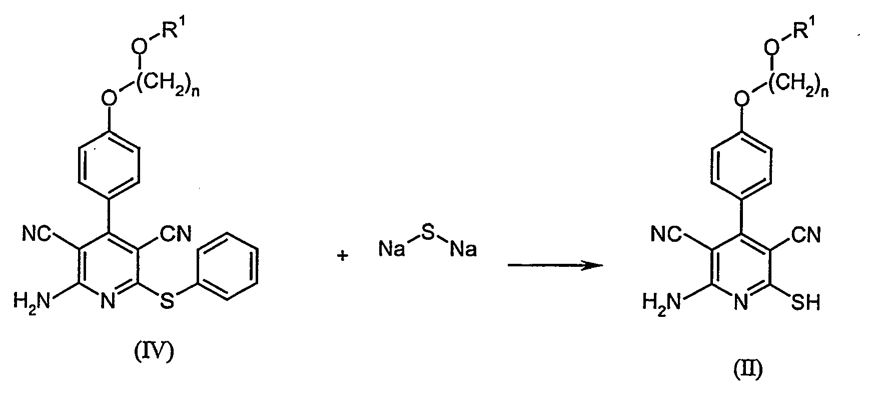

Als Alkalisulfid wird vorzugsweise Natriumsulfid in einer Menge von 1 bis 10 Mol, bevorzugt 1 bis 5 Mol, insbesondere 1 bis 4 Mol, bezogen auf 1 Mol der Verbindungen der Formel (IV) eingesetzt.

Als Lösungsmittel geeignet sind alle organischen Lösungsmittel, die unter den Reaktionsbedingungen inert sind. Hierzu gehören beispielsweise N,N-Dimethylformamid, N-Methylpyrrolidinon, Pyridin und Acetonitril. Bevorzugt ist N,N-Dimethylformamid. Ebenso ist es möglich, Gemische der zuvor genannten Lösungsmittel einzusetzen.

Die Reaktion erfolgt im allgemeinen in einem Temperaturbereich von +20°C bis +140°C, bevorzugt im Bereich von +20°C bis +120°C, insbesondere bei +60°C bis +100°C.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Die Verbindungen der Formel (III) sind entweder kommerziell erhältlich, dem Fachmann bekannt oder nach üblichen Methoden herstellbar.

Die Verbindungen der Formel (IV) sind entweder kommerziell erhältlich, dem Fachmann bekannt oder nach üblichen Methoden herstellbar. Insbesondere kann auf die folgenden Druckschriften verwiesen werden, deren jeweiliger Inhalt durch Bezugnahme eingeschlossen wird:
● Kambe et al., Synthesis, 531 bis 533 (1981);
● Elnagdi et al., Z. Naturforsch.47b, 572 bis 578 (1991).

Die pharmazeutische Wirksamkeit der Verbindungen der Formel (I) lässt sich durch ihre Wirkung als selektiver Ligand an Adenosin-A1 Rezeptoren erklären. Sie wirken hierbei als A1-Agonisten.

Überraschenderweise zeigen die Verbindungen der Formel (I) ein nicht vorhersehbares, wertvolles pharmakologisches Wirkspektrum und sind daher insbesondere zur Prophylaxe und/oder Behandlung von Erkrankungen geeignet.

Gegenüber dem Stand der Technik verfügen die erfindungsgemäßen Verbindungen der Formel (I) über verbesserte pharmakokinetische Eigenschaften, insbesondere über eine bessere Bioverfügbarkeit nach oraler Gabe.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere von Erkrankungen des Herzkreislaufsystems (kardiovaskulären Erkrankungen). Geeignete Kombinationswirkstoffe sind insbesondere Wirkstoffe zur Behandlung von koronaren Herzkrankheiten wie beispielsweise insbesondere Nitrate, Betablocker, Calciumantagonisten oder Diuretika.

Im Sinne der vorliegenden Erfindung sind unter Erkrankungen des HerzkreislaufSystems bzw. kardiovaskulären Erkrankungen beispielsweise insbesondere die folgenden Erkrankungen zu verstehen: Koronare Restenose wie z.B. Restenose nach Ballondilatation von peripheren Blutgefäßen, Tachykardien, Arrhythmien; periphere und kardiale Gefäßerkrankungen, stabile und instabile Angina pectoris und Vorhof- und Kammerflimmern.

Weiterhin eignen sich die Verbindungen der Formel (I) beispielsweise insbesondere auch zur Reduktion des von einem Infarkt betroffenen Myokardbereichs.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Weitere Indikationsgebiete, für das sich die Verbindungen der Formel (I) eignen, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündlichen Dermatosen, von neuroinflammatorischen Erkrankungen des Zentralnervensystems, wie beispielsweise Zustände nach Hirninfarkt, der Alzheimer-Erkrankung, weiterhin auch von neurodegenerative Erkrankungen, sowie von Schmerzzuständen und Krebs.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasien, zystische Fibrose (Mukoviszidose) und pulmonale Hypertonie.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Verbindungen der Formel (I).

Weiterer Gegenstand der vorliegenden Erfindung sind Arzneimittel, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal wie z.B. bei Implantaten oder Stents, oder äußerlich wie z.B. transdermal. Bei der parenteralen Applikation sind insbesondere intravenöse, intramuskuläre, subkutane Applikation zu nennen, z.B. als subkutanes Depot. Bevorzugt ist die orale oder parenterale Applikation. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen. Insbesondere sollte die Konzentration des Wirkstoffs 0,5 bis 90 Gew.-% betragen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten selbstverständlich auch Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Im allgemeinen hat es sich als vorteilhaft erwiesen, bei parenteraler Applikation Mengen von etwa 0,1 bis etwa 10.000 µg/kg, vorzugsweise etwa 1 bis etwa 1.000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,05 bis etwa 5 mg/kg, vorzugsweise etwa 0,1 bis etwa 5 mg/kg, insbesondere etwa 0,1 bis etwa 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die vorliegende Erfindung wird an den folgenden, nicht einschränkenden bevorzugten Beispielen veranschaulicht, die die Erfindung jedoch keinesfalls beschränken.

Die Prozentangaben der nachfolgenden Beispiele beziehen sich, sofern nicht anders angegeben, jeweils auf das Gewicht; Teile sind Gewichtsteile.

### A. Bewertung der physiologischen Wirksamkeit

### I. Nachweis der kardiovaskulären Wirkung

Narkotisierten Ratten wird nach Eröffnung des Brustkorbes das Herz schnell entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

In dieser Versuchsanordnung wurden folgende Werte für die Senkung der Herzfrequenz erhalten (der prozentual angegebene Wert bezieht sich auf die prozentuale Absenkung der Herzfrequenz bei der jeweiligen Konzentration):

| Verbindung aus Beispiel | Prozentuale Absenkung der Herzfrequenz bei einer Konzentration von | |
|---|---|---|
| | 10⁻⁷ g/ml | 10⁻⁶ g/ml |
| 1 | 15,0 % | 17,5 % |
| 6 | 15,5 % | 20,0 % |

### II. Bestimmung des Adenosin-A1-, A2a-, A2b- und A3-Agonismus

### a) Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a, A2b transfiziert. Die Adenosin A1 Rezeptoren sind über Gᵢ-Proteine und die Adenosin A2a und A2b Rezeptoren über Gs-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanztest-Screening wird das folgende Testprotokoll verwendet:

Die Stammkulturen werden in DMEM/F12 Medium mit 10 % FCS (fötales Kälberserum) bei 37°C unter 5 % CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid 6 H₂O, 5 mM NaHCO₃, pH 7,4) ersetzt. Die in DMSO gelösten Substanzen werden dreimal 1:10 mit dieser physiologischen Kochsalzlösung verdünnt und zu den Testkulturen pipettiert (maximale DMSO-Endkonzentration im Testansatz: 0,5 %). So erhält man Substanzendkonzentrationen von beispielsweise 5 µM bis 5 nM. 10 Minuten später wird Forskolin zu den A1 Zellen zugegeben und anschließend werden alle Kulturen für vier Stunden bei 37°C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50 % aus Luciferase Substrat Lösung (2,5 mM ATP, 0,5 mM Luciferin, 0,1 mM Coenzym A, 10 mM Tricin, 1,35 mM Magnesiumsulfat, 15 mM DTT, pH 7,8) zugegeben, ca. 1 Minute geschüttelt und die Luciferase-Aktivität mit einem Kamerasystem gemessen. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

In der folgenden Tabelle 1 sind Werte für die Rezeptorstimulation der Verbindung aus Beispiel 1 und 6 bei verschiedenen Konzentrationen an verschiedenen Adenosin-Rezeptor Subtypen angegeben.

**Tabelle 1: Adenosin-Rezeptorstimulation der Verbindungen aus Beispiel 1 und 6 bei verschiedenen Konzentrationen**

| Rezeptorsubtyp | Beispiel 1 | | | Beispiel 6 | | |
|---|---|---|---|---|---|---|
| | 10 nmol | 1 nmol | 0,3 nmol | 10 nmol | 1 nmol | 0,3 nmol |
| A1 | 4% | 11% | 56% | 7% | 25% | 45% |
| A2a | -2% | 2% | -1 % | 2% | 4%. | 0% |
| A2b | 8% | 6% | 2% | 29% | 3% | 0 |

Angegeben sind die %-Werte des entsprechenden Referenzstimulus. Die Messwerte für den A2a- und den A2b-Rezeptor sind Angaben in Prozent der maximalen Stimulation durch NECA; die Messwerte für den A1-Rezeptor sind Angaben in Prozent nach direkter Vorstimulation der Adenylatcyclase durch 1 µmolar Forskolin (entspricht 100 %-Wert). A1-Agonisten zeigen entsprechend eine Abnahme der Luciferase-Aktivität (Messwert kleiner 100 %).

### b) Direkte Bestimmung des Adenosin-Agonismus über cAMP-Nachweis

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des intrazellulären cAMP-Gehaltes in diesen Zellen mit einem konventionellen radioimmunologischen Assay (cAMP-RIA, IBL GmbH, Hamburg, Deutschland).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die nicht selektiv, aber mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

Die Adenosin-Rezeptoren A1 und A3 sind an ein Gᵢ-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), das mit hoher Affinität an alle Adenosinrezeptor-Subtypen bindet und eine antagonistische Wirkung besitzt (Müller, C.E., Stein, B., Adenosine receptor antagonists: Structures and potential therapeutic applications, Current Pharmaceutical Design, 2 (1996), 501-530).

### III. Pharmokokinetische Untersuchungen

Pharmakokinetische Daten wurden nach i.v. sowie nach p.o. Gabe verschiedener Substanzen als Lösung an Mäusen, Ratten und Hunden ermittelt. Hierzu wurden bis 24 h nach Applikation Blutproben gesammelt. In den daraus gewonnenen Plasmaproben wurden die Konzentrationen der unveränderten Substanz mittels bioanalytischer Methoden (HPLC oder HPLC-MS) bestimmt. Anschließend wurden aus den so erhaltenen Plasmakonzentrations-Zeitverläufen pharmakokinetische Parameter ermittelt. In der folgenden Tabelle 2 sind die Bioverfiigbarkeiten bei verschiedenen Spezies angegeben.

**Tabelle 2: Bioverfügbarkeiten nach oraler Gabe**

| | Maus | Ratte | Hund |
|---|---|---|---|
| Beispiel 22 in WO/01/25210 | nicht bestimmbar* (bei 3 mg/kg p.o) | nicht bestimmbar* (bei 10 mg/kg p.o) | 1,47 % (bei 1 mg/kg p.o) |
| Verbindung aus Beispiel 1 | 31,5 % (bei 1 mg/kg p.o) | 5,0 % (bei 3 mg/kg p.o) | 32,6 % (bei 3 mg/kg p.o) |
| Verbindung aus Beispiel 6 | 41,3 % (bei 3 mg/kg p.o) | 42,3 % (bei 3 mg/kg p.o) | 28,5 % (bei 1 mg/kg p.o) |

| | | | |
|---|---|---|---|
| * Plasmaspiegel zu allen Messzeitpunkten unterhalb der Bestimmungsgrenze (<1 µg/l) | | | |

### B. Ausführungsbeispiele

### Verwendete Abkürzungen:

- DBU: 1,8-Diazabicyclo[5.4.0]undec-7-en
- DMF: Dimethylformamid
- ESI: Elektrospray-Ionisation (bei MS)
- HEPES: 2-[4-(2-Hydroxyethyl)piperazino]ethansulfonsäure
- HPLC: Hochdruck-, Hochleistungsflüssigkeitschromatographie
- Kp.: Siedepunkt
- MS: Massenspektroskopie
- NMR: Kernresonanzspektroskopie
- p.A.: pro analysi
- RT: Raumtemperatur
- i.V.: im Vakuum
- d.Th.: der Theorie (bei Ausbeute)
- Tris: 2-Amino-2-(hydroxymethyl)-1,3-propandiol

### Herstellungsbeispiele

### Beispiel 1

### 2-Amino-4-[4-(2-methoxyethoxy)phenyl]-6-[(3-pyridinylmethyl)sulfanyl]-3,5-pyridindicarbonitril

### 1. Stufe:

### 4-(2-Methoxyethoxy)benzaldehyd

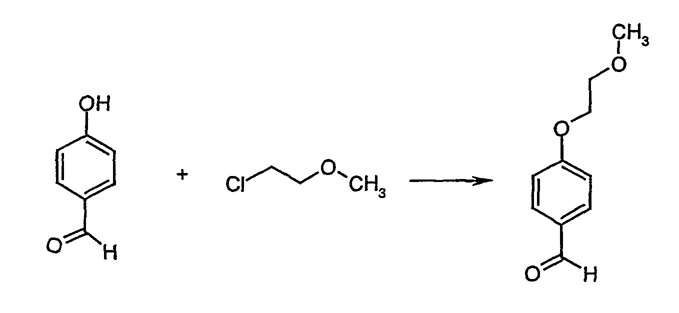

146.5 g (1.2 Mol) 4-Hydroxybenzaldehyd werden in DMF gelöst und mit 20 g (0.12 Mol) Kaliumiodid, 134.6 g (1.2 Mol) Kalium-tert.-butylat und 170.2 g (1.8 Mol) (2-Chlorethyl)methylether versetzt. Die Reaktionsmischung wird 16 h bei 80°C gerührt. Zur Aufarbeitung wird die Reaktionsmischung im Vakuum eingeengt. Der Rückstand wird in 11 Ethylacetat aufgenommen und mit 0.5 11 N Natronlauge extrahiert. Die Ethylacetat-Phase wird mit Magnesiumsulfat getrocknet und i.V. eingedampft. Der Eindampfrückstand wird im Hochvakuum destilliert (Kp. = 100°C bei 0.45 mbar). Man erhält 184.2 g (85 % d.Th.) Produkt.
MS (ESIpos): m/z =181 (M+H)⁺
¹H-NMR (300 MHz, CDCl₃): δ = 3.5 (s, 3H); 3.8 (tr, 2H); 4.2 (tr, 2H); 7.0 (d, 2H); 7.8 (d, 1H); 9.9 (s, 1H).

### 2. Stufe:

### 2-Amino-4-[4-(2-methoxyethoxy)phenyl]-6-sulfanyl-3,5-pyridindicarbonitril

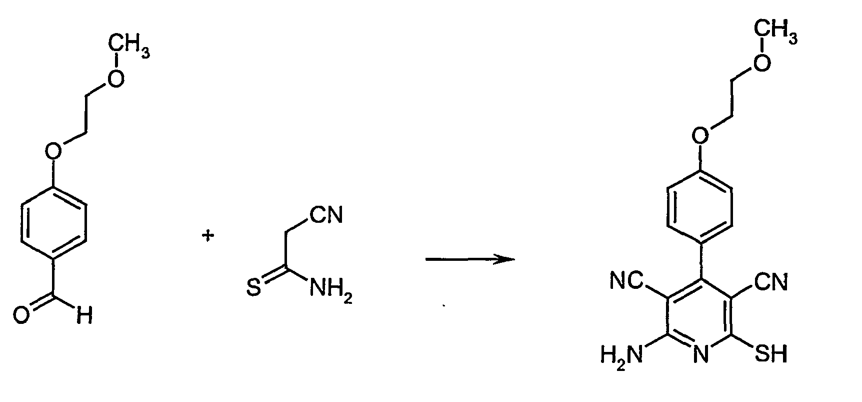

18 g (100 mmol) 4-(2-Methoxyethoxy)benzaldehyd, 10 g (200 mmol) Cyanthioacetamid und 20.2 g (200 mmol) N-Methylmorpholin werden in 100 ml Ethanol 3 h unter Rückfluss erhitzt. Nach Abkühlen werden die ausgefallenen Kristalle abgesaugt, mit wenig Ethanol gewaschen und i.V. getrocknet. Man erhält 12 g (3I% d.Th.) Produkt, das 0.5 Molequivalent N-Methylmorpholin enthält.
MS (ESIpos): m/z = 327 (M+H)⁺
¹H-NMR (300 MHz, DMSO-d₆): δ = 2.8 (tr, 4H, N-Methylmorpholinsignal); 3.3 (s, 3H); 3.7 (m, 2H, + 4H N-Methyhnorpholinsignal); 4.2 (tr, 2H); 7.1 (d, 2H); 7.4 (d, 2H); 7.6 (s, breit, 2H).

### 3. Stufe:

### 2-Amino-4-[4-(2-methoxyethoxy)phenyl]-6-[(3-pyridinylinethyl)sulfanyl]-3,5-pyridindicarbonitril

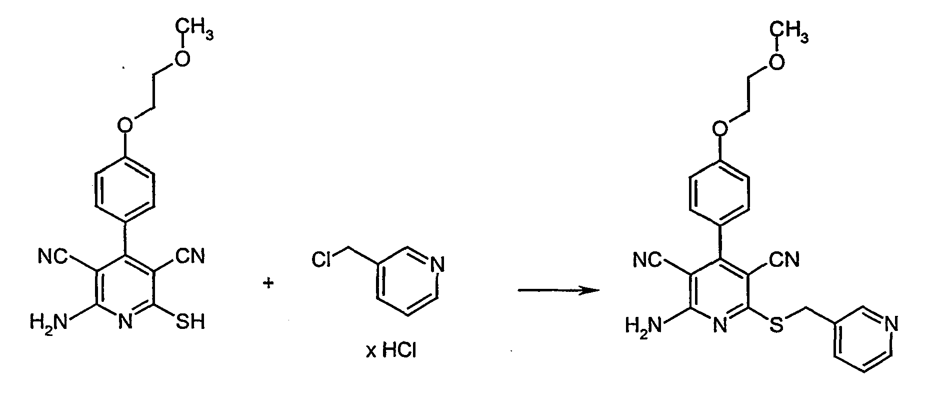

4.28 g (11.36 mmol; das Edukt enthielt 0.5 Molequivalent N-Methylmorpholin, daher 86.6 % Reinheit) 2-Amino-4-[4-(2-methoxyethoxy)phenyl]-6-sulfanyl-3,5-pyridindicarbonitril werden in 40 ml DMF p.A. gelöst. Dann werden 3.34 g (39.75 mmol) Natriumhydrogencarbonat und 2.48 g (15.1 mmol) 3-Picolylchlorid-Hydrochlorid zugegeben. Die Suspension wird über Nacht bei RT gerührt, mit 40 ml Ethanol versetzt und auf ca. 40°C erwärmt. Dann werden 19 ml Wasser zugetropft. Der Niederschlag wird abgesaugt und i.V. getrocknet. Man erhält 3.70 g (78 % d.Th.) Produkt.
MS (ESIpos): m/z = 418 (M+H)⁺
¹H-NMR (300 MHz, DMSO-d₆): δ = 3.3 (s, 3H); 3.7 (tr, 2H); 4.2 (tr, 2H); 4.5 (s, 2H); 7.1 (d, 2H); 7.35 (dd, 1H); 7.45 (d, 2H); 7.9 (d tr, 1H); 8.1 (s, breit, 2H); 8.45 (dd, 1H); 8.75 (d, 1H).

### Beispiel 2

### 2-Amino-6-[(2-chlor-1,3-thiazol-4-yl)methylsulfanyl]-[4-(2-methoxyethoxy)phenyl]-3,5-pyridindicarbonitril

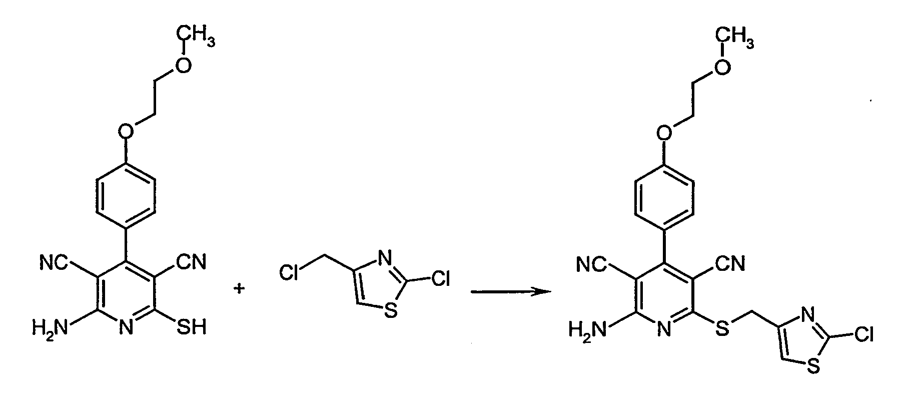

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 77.2 mg (0.46 mmol) 4-Chlormethyl-2-chlor-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 123 mg (88 % d.Th.) Produkt.
MS (ESIpos): m/z = 458 (M+H)⁺
¹H-NMR (300 MHz, DMSO-d₆): δ = 3.3 (s, 3H); 3.7 (tr, 2H); 4.2 (tr, 2H); 4.5 (s, 2H); 7.1 (d, 2H); 7.45 (d, 2H); 7.8 (s, 1H); 8.05 (s, breit, 2H).

### Beispiel 3

### 2-Amino-4-[4-(2-methoxyethoxy)phenyl]-6-[(2-phenyl-1,3-thiazol-4-yl)methylsulfanyl]-3,5-pyridindicarbonitril

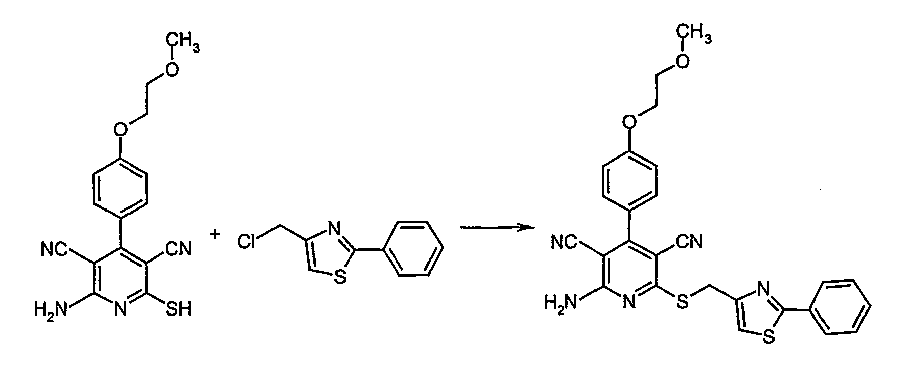

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 96.4 mg (0.46 mmol) 4-Chlormethyl-2-phenyl-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 149 mag (97 % d.Th.) Produkt.
MS (ESIpos): m/z = 500 (M+H)⁺
¹H-NMR (300 MHz, DMSO-d₆): δ = 3.3 (s, 3H); 3.7 (tr, 2H); 4.2 (tr, 2H); 4.5 (s, 2H); 7.1 (d, 2H); 7.5 (m, 5R); 7.8 (s, 1H); 7.9 (m, 2H); 8.05 (s, breit, 2H).

### Beispiel 4

### 2-Amino-4-[4-(2-methoxyethoxy)phenyl]-6-[(2-(thiophen-2-yl)-1,3-thiazol-4-yl)-methylsulfanyl]-3,5-pyridindicarbonitril

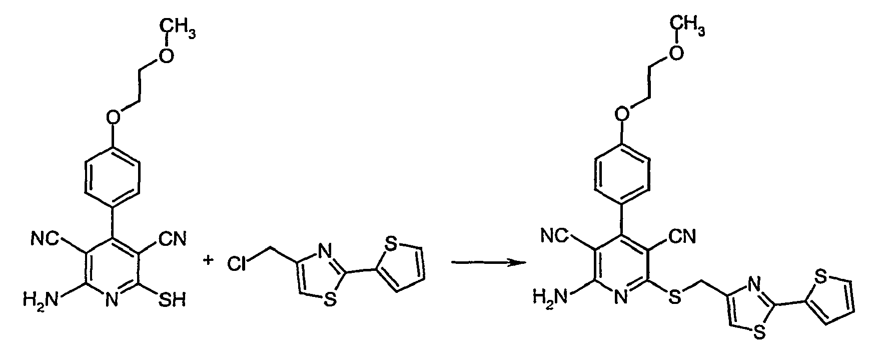

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 96.4 mg (0.46 mmol) 4-Chlormethyl-2-(thiophen-2-yl)-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 146 mg (84 % d.Th.) Produkt.
MS (ESIpos): m/z = 506 (M+H)⁺
¹H-NMR (300 MHz, DMSO-d₆): δ = 3.3 (s, 3H); 3.7 (tr, 2H); 4.2 (tr, 2H); 4.6 (s, 2H); 7.15 (m, 3H); 7.5 (d, 2H); 7.65 (d, 1H); 7.75 (d , 1H); 7.8 (s, 1H); 8.1 (s, breit, 2H).

### Beispiel 5

### 2-Amino-4-[4-(2-methoxyethoxy)phenyl]-6-[(2-(thiophen-3-yl)-1,3-thiazol-4-yl)-methylsulfanyl] -3,5-pyridindicarbonitril

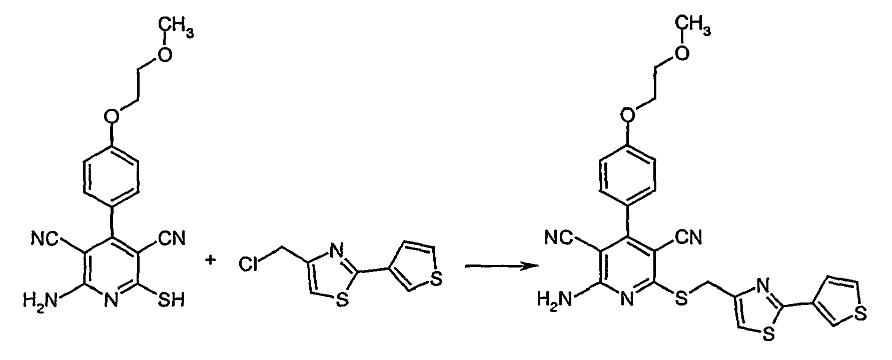

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 96.4 mg (0.46 mmol) 4-Chlormethyl-2-(thiophen-3-yl)-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 141 mg (82 % d.Th.) Produkt.
MS (ESIpos): m/z = 506 (M+H)⁺
¹H-NMR (300 MHz, DMSO-d₆): δ = 3.3 (s, 3H); 3.7 (tr, 2H); 4.2 (tr, 2H); 4.6 (s, 2H); 7.15 (d, 2H); 7.5 (d, 2H); 7.55 (d, 1H); 7.7 (dd, 1H); 7.8 (s, 1H); 8.1 (s, breit, 2H); 8.15 (d, 1H).

### Beispiel 6

### 2-Amino-6-({[2-(4-chlorphenyl)-1,3-thiazol-4-yl]methyl}sulfanyl)-4-[4-(2-hydroxyethoxy)phenyl]-3,5-pyridindicarbonitril

### Weg 1:

### 1. Stufe:

### 2-Amino-4-[4-(2-hydroxyethoxy)phenyl]-6-sulfanyl-3,5-pyridindicarbonitril

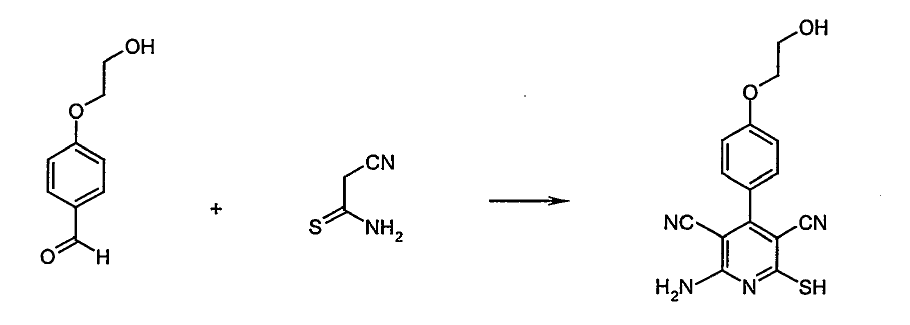

12.46 g (75 mmol) 4-(2-Hydroxyethoxy)benzaldehyd, 15.02 g (150 mmol) Cyanthioacetamid und 15.15 g (150 mmol) N-Methyltnorpholin werden in 75 ml Ethanol vorgelegt und 3 h unter Rückfluss erhitzt. Die Reaktionslösung wird nach Abkühlen i.V. eingedampft. Der Rückstand wird in 1 N Natronlauge gelöst und zweimal mit Essigsäureethylester gewaschen. Die Natronlauge-Phase wird mit 1 N Salzsäure angesäuert, die ausgefallenen Kristalle werden abgesaugt und i.V. bei 45°C getrocknet. Man erhält 12.05 g (51% d.Th.) Produkt.
MS (ESIpos): m/z = 313 (M+H)⁺, 330 (M+NH₄)⁺
¹H-NMR (300 MHz, DMSO-d₆): δ = 3.7 (t, 2H); 4.1 (t, 2H); 7.1 (d, 2H); 7.4 (d, 2H); 8.0 (br s, 2H).

### 2. Stufe:

### 2-Ainino-6-({[2-(4-chlorphenyl)-1,3-thiazol-4-yl]methyl}sulfanyl)-4-[4-(2-hydroxyethoxy)phenyl]-3,5-pyridindicarbonitril

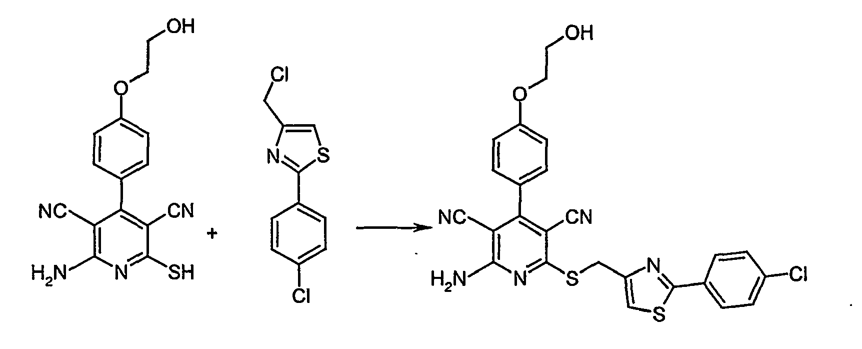

6.91 g (22.12 mmol) 2-Amino-4-[4-(2-hydroxyethoxy)phenyl]-6-sulfanyl-3,5-pyridindicarbonitril werden in 150 ml DMF gelöst. Dann werden 7.44 g (66.35 mmol) 1,8-Diazabicyclo[5.4.0]undec-7-en und 10.8 g (44.24 mmol) 4-Chlormethyl-2-(4-chlorphenyl)-1,3-thiazol zugegeben. Die Suspension wird über Nacht bei RT gerührt, mit 50 g Kieselgel versetzt und i.V. eingedampft. Das auf dem Kieselgel aufgezogene Substanzgemisch wird durch Chromatographie an Kieselgel (Elutionsmittel: Toluol bis Toluol/Essigsäureethylester 1:1-Gemisch) gereinigt. Man erhält 5.5 g (47 % d.Th.) Produkt.
MS (ESIpos): m/z = 521 (M+H)⁺
¹H-NMR (300 MHz, DMSO-d₆): δ = 3.7 (dt, 2H); 4.1 (t, 2H); 4.6 (s, 2H); 4.9 (t, 1H); 7.1 (d, 2H); 7.4 (d, 2H); 7.5 (d, 2H); 7.9 (m, 3H); 8.1 (br s, 2H).

### Weg 2:

Alternativ kann die Herstellung auch ohne Isolierung von 2-Amino-4-[4-(2-hydroxyethoxy)phenyl]-6-sulfanyl-3,5-pyridindicarbonitril durch Umsetzung von 2-[4-(2-Hydroxyethoxy)-benzyliden]malononitril mit 2-Cyanthioacetamid und 4-Chlormethyl-2-(4-chlorphenyl)-1,3-thiazol erfolgen:

### 1. Stufe:

### 2-[4-(2-Hydroxyethoxy)-benzyliden]malononitril

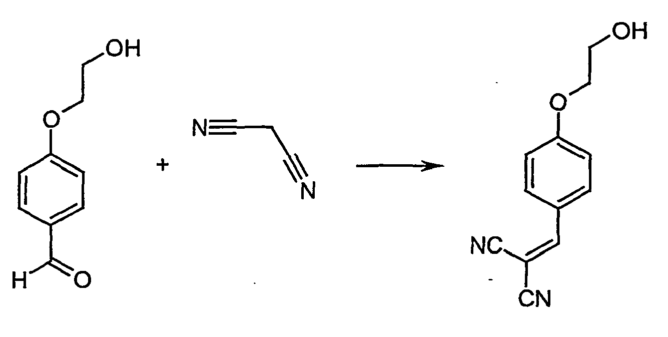

1000 g (5.85 mol) 4-(2-Hydroxyethoxy)benzaldehyd und 425 g (6.43 mol) Malondinitril werden in 5000 ml Isopropylalkohol gelöst und mit 5 g (0.059 mol) Piperidin versetzt. Die Mischung wird für 16 Stunden auf 80°C erhitzt und zur Isolierung des Produktes nachfolgend auf 3°C gekühlt. Das Produkt wird abfiltriert und mit 400 ml eiskaltem Isopropylalkohol gewaschen. Nachfolgend wird im Vakuum (40 mbar) für 45 Stunden bei 50°C getrocknet.
Ausbeute: 1206 g (94.6 % d.Th.) leicht gelbe Kristalle
¹H (400 MHz, CDCl₃): 3.95 - 4.32 m (4 H), 6.95 - 7.15 (m, 2H), 7.61 (s, 1H), 7.85 - 7.95 (m, 1H).

### 2. Stufe:

### 4-Chlormethyl-2-(4-chlorphenyl)-1,3-thiazol

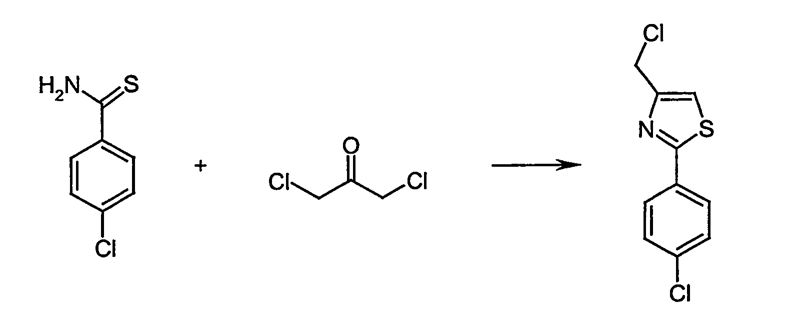

171.65 g (1.0 mol) 4-Chlorthiobenzamid werden in 550 ml Isopropylalkohol gelöst und innerhalb von 3 Stunden bei maximal 30°C mit 133.3 g (1.05 mol) 1,3-Dichloraceton versetzt. Man rührt für 5.5 Stunden bei 40°C und 10 Stunden bei 20°C nach. Zur Vervollständigung der Reaktion wird nun für 7.5 Stunden auf 55°C erwärmt. Zur Isolierung des Produktes wird auf 10°C gekühlt und mit 950 ml Wasser versetzt. Der pH-Wert wird dabei mit Natronlauge auf 4 bis 5 eingestellt und das Produkt abgesaugt.
Ausbeute: 220.9 g (91 % d.Th.) weiße bis leicht gelbe Kristalle
¹H (400 MHz, CDCl₃): 4.90 (s, 2H, CH₂), 7.5 - 7.55 (m, 2H), 7.85 (s, 1H, Thiazol), 7.9 -7.95 (m, 2H)

### 3. Stufe:

### 2-Amino-6-({[2-(4-chlorphenyl)-1,3-thiazol-4-yl]methyl}sulfanyl)-4-[4-(2-hydroxyethoxy)phenyl]-3, S-pyridindicarbonitril

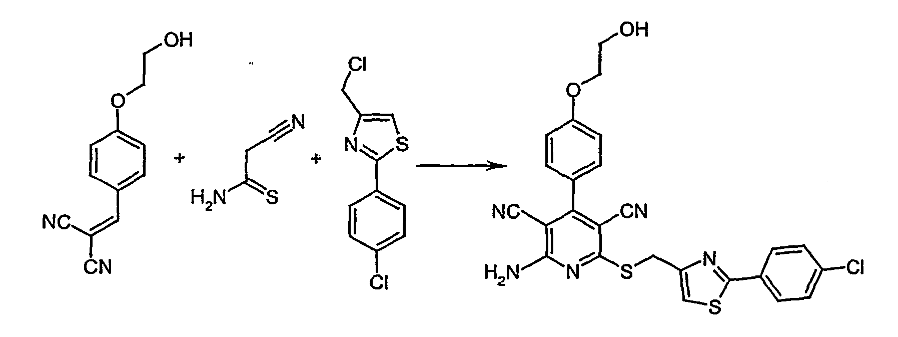

428.4 g (2.0 mol) 2-[4-(2-Hydroxyethoxy)-benzyliden]malononitril, 108.4 g (1.05 mol) 2-Cyanthioacetamid und 244.1 g (1.0 mol) 4-Chlormethyl-2-(4-chlorphenyl)-1,3-thiazol werden in 3.4 Litern Methanol suspendiert und innerhalb von 60 Minuten mit 556.1 g (3.0 mol) Tributylamin versetzt. Man rührt für 20 Stunden bei Raumtemperatur nach und filtriert das Produkt ab. Nach dem Trocknen im Vakuum wird das Rohprodukt (360.8 g, Rohausbeute 70 % d.Th.) in 3 Litern Dichlormethan suspendiert und bei 35°C für 2 Stunden gerührt. Das Produkt wird abfiltriert und im Hochvakuum getrocknet. Die nunmehr weißen Kristalle können zur weiteren Aufreinigung aus Tetrahydrofuran/Wasser (1:1) umkristallisiert werden.
Ausbeute: 353.5 g (68 % d.Th.) weiße Kristalle
MS(EI): m/z= 520.00

### Beispiel 7

### 2-Amino-4-[4-(2-methoxyethoxy)phenyl]-6-[(2-pyridinylmethyl)sulfanyl]-3,5-pyridindicarbonitril

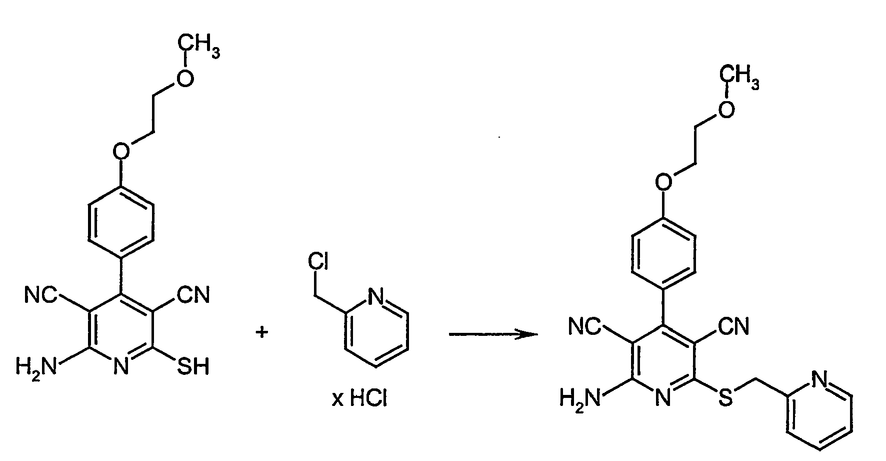

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 75.4 mg (0.46 mmol) 2-Picolylchlorid-Hydrochlorid zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 104 mg (81% d.Th.) Produkt.
MS (ESIpos): m/z = 418 (M+H)⁺
¹H-NMR (300 MHz, DMSO-d₆): δ = 3.3 (s, 3H); 3.7 (tr, 2H); 4.2 (tr, 2H); 4.6 (s, 2H); 7.1 (d, 2H); 7.4 (dd, 1H); 7.45 (d, 2H); 7.65 (d, 1H); 7.75 (tr, 1H); 8.0 (s, breit, 2H); 8.5 (d, 1H).

### Beispiel 8

### 2-Amino-4-[4-(2-methoxyethoxy)phenyl]-6-[(2-methyl-1,3-thiazol-4-yl)methyl-sulfanyl]-3,5-pyridindicarbonitril

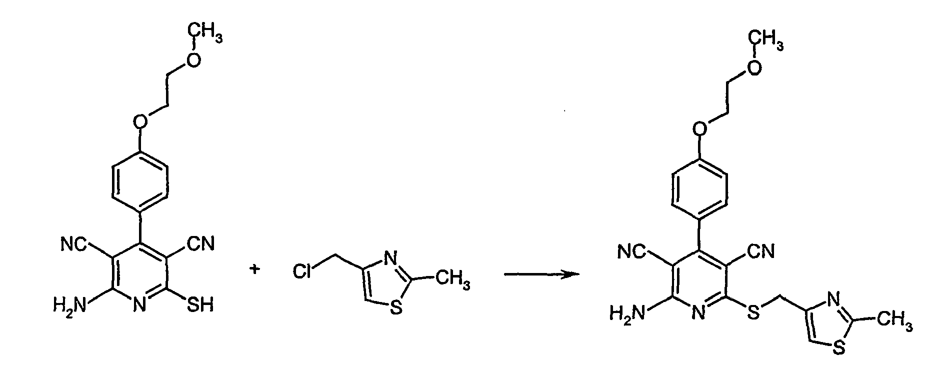

100 mg (0.31 mmol) 2-Amino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 90.5 mg (0.61 mmol) 4-Chlormethyl-2-methyl-1,3-thiazol zugegeben. Die Suspension wird über Nacht bei RT geschüttelt und mit Wasser versetzt. Der Niederschlag wird abgesaugt und bei 40°C i.V. getrocknet. Man erhält 88.8 mg (66.2 % d.Th.) Produkt.
MS (ESIpos): m/z = 438 (M+H)⁺

### Beispiel 9

### 2-Amino-4-[4-(2-methoxyethoxy)phenyl]-6-[(2-amino-1,3-thiazol-4-yl)methyl-sulfanyl]-3,5-pyridindicarbonitril

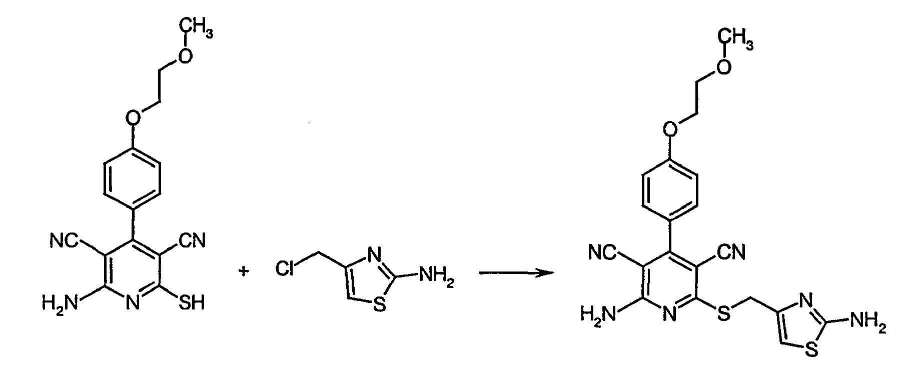

100 mg (0.31 mmol) 2-Axnino-4-[4-(2-methoxyethoxy)phenyl]-6-sulfanyl-3,5-pyridindicarbonitril werden in 1 ml DMF gelöst. Dann werden 103 mg (1.23 mmol) Natriumhydrogencarbonat und 68.3 mg (0.46 mmol) 4-Chlormethyl-2-amino-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 115.9 mg (86.2 % d.Th.) Produkt.
MS (ESIpos): m/z = 439 (M+H)⁺

### Beispiel 10

### 2-Amino-4-[4-(2-methoxyethoxy)phenyl]-6-[(2-(2-pyridyl)-1,3-thiazol-4-yl)methyl-sulfanyl]-3,5-pyridindicarbonitril

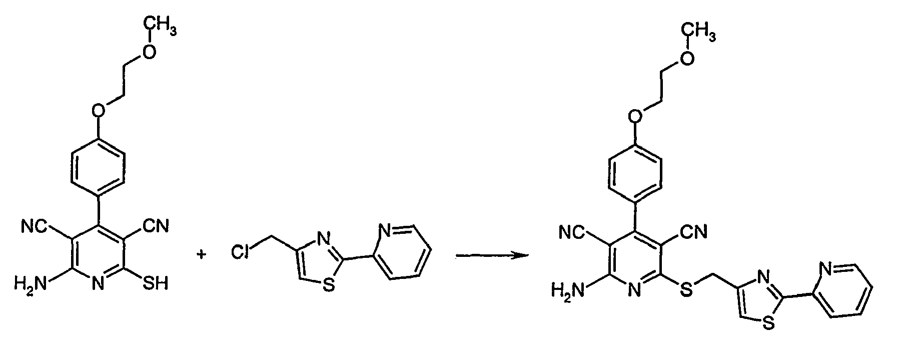

50 mg (0.15 mmol) 2-Amino-4-[4-(2-methoxyethoxy)phenyl]-6-sulfanyl-3,5-pyridindicarbonitril werden in 1 ml DMF gelöst. Dann werden 51.5 mg (0.61 mmol) Natriumhydrogencarbonat und 58.6 mg (0.23 mmol) 4-Chlormethyl-2-(2-pyridyl)-1,3-thiazol zugegeben. Die Suspension wird über Nacht bei RT geschüttelt und mit Wasser versetzt. Der Niederschlag wird abgesaugt, mit Ethanol und Diethylether gewaschen und bei 40°C i.V. getrocknet. Man erhält 67.4 mg (87.9 % d.Th.) Produkt.
MS (ESIpos): m/z = 501 (M+H)⁺

### Beispiel 11

### 2-Amino-4-[4-(2-hydroxyethoxy)phenyl]-6-{[(2-methyl-1,3-thiazol-4-yl)methyl]-sulfanyl}-3,5-pyridindicarbonitril

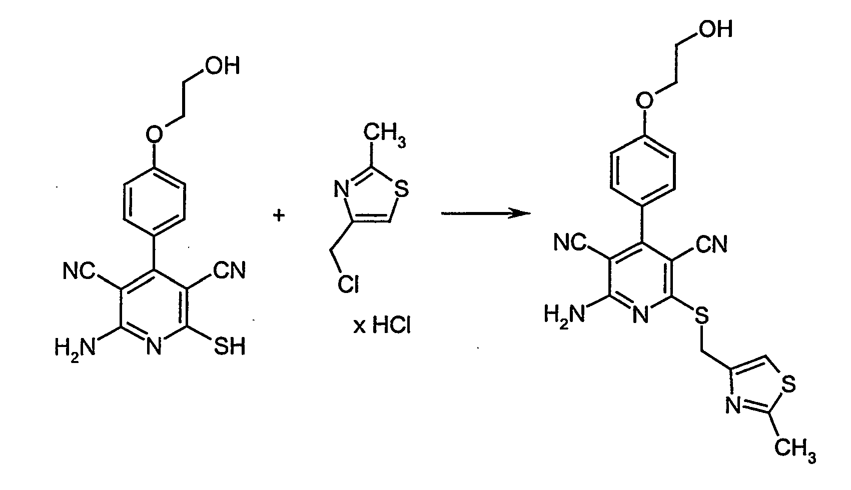

31.2 mg (0.1 mmol) 2-Amino-4-[4-(2-hydroxyethoxy)phenyl]-6-sulfanyl-3,5-pyridindicarbonitril werden in 0.3 ml DMF gelöst. Dann werden 33.6 mg (0.4 mmol) Natriumhydrogencarbonat und 27.6 mg (0.15 mmol) 4-Methyl-2-chlor-1,3-thiazol-Hydrochlorid zugegeben. Die Suspension wird über Nacht bei RT geschüttelt, filtriert und durch präparative HPLC gereinigt [Säule: Macherey-Nagel VP 50/21 Nucleosil 100-5 C18 Nautilus, 20 x 50 mm; Flussrate: 25 ml/min; Gradient (A = Acetonitril, B = Wasser + 0.3 % Trifluoressigsäure): 0 min 10 % A; 2.0 min 10 % A; 6.0 min 90 % A; 7.0 min 90 % A; 7.1 min 10 % A; 8.0 min 10 % A; Detektion: 220 nm]. Nach Eindampfen der entsprechenden Fraktion erhält man 20.2 mg (47.7 % d.Th.) Produkt.
MS (ESIpos): m/z = 424 (M+H)⁺

### Beispiel 12

### 2-Amino-6-{[(2-amino-1,3-thiazol-4-yl)methyl]sulfanyl}-4-[4-(2-hydroxyethoxy)-phenyl]-3,5-pyridindicarbonitril

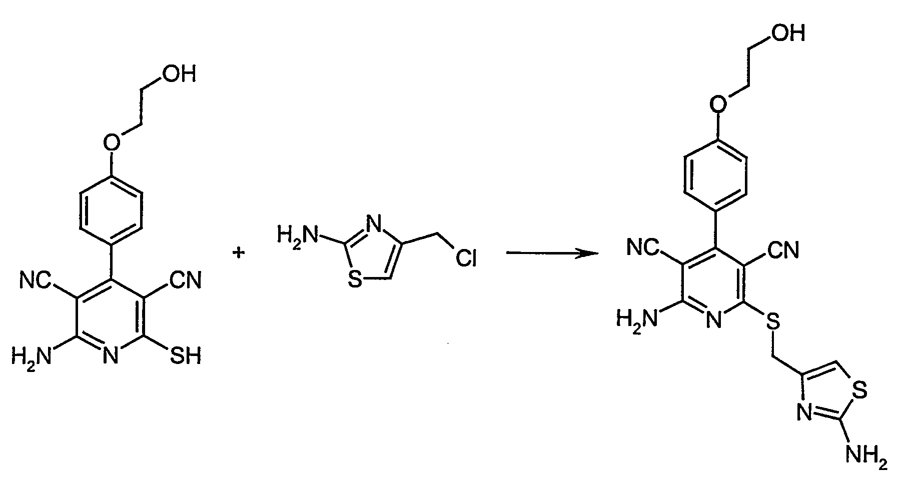

31.2 mg (0.1 mmol) 2-Amino-4-[4-(2-hydroxyethoxy)phenyl]-6-sulfanyl-3,5-pyridindicarbonitril werden in 0.3 ml DMF gelöst. Dann werden 33.6 mg (0.4 mmol) Natriumhydrogencarbonat und 22.3 mg (0.15 mmol) 4-Amino-2-chlor-1,3-thiazol zugegeben. Die Suspension wird über Nacht bei RT geschüttelt, filtriert und durch präparative HPLC gereinigt [Säule: Macherey-Nagel VP 50/21 Nucleosil 100-5 C18 Nautilus, 20 x 50 mm; Flussrate: 25 ml/min; Gradient (A = Acetonitril, B = Wasser + 0.3 % Trifluoressigsäure): 0 min 10 % A; 2.0 min 10 % A; 6.0 min 90 % A; 7.0 min 90 % A; 7.1 min 10 % A; 8.0 min 10 % A; Detektion: 220 nm]. Nach Eindampfen der entsprechenden Fraktion erhält man 35.7 mg (84.1 % d.Th.) Produkt.
MS (ESIpos): m/z = 425 (M+H)⁺

### Beispiel 13

### 2-Amino-4-[4-(2-methoxyethoxy)phenyl]-6-({[2-(4-morpholinyl)-1,3-thiazol-4-yl]-methyl} sulfanyl)-3,5-pyridindicarbonitril

### 1. Stufe:

### 4-[4-(Chlormethyl)-1,3-thiazol-2-yl]morpholin

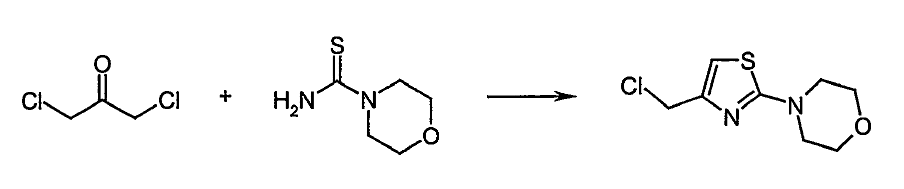

11.51 g (78.76 mmol) 4-Morpholincarbothioamid und 10.00 g (78.76 mmol) Dichloraceton werden in 100 ml Ethanol eine Stunde unter Rückfluss erhitzt. Der aus der pink-farbenen Lösung ausfallende farblose Feststoff wird nach dem Abkühlen abgesaugt und zweimal mit Ethanol gewaschen. Man erhält 12.96 g (75 % d.Th.) Produkt.
MS (ESIpos): m/z = 219 (M+H)⁺

### 2. Stufe:

### 2-Amino-4-[4-(2-methoxyethoxy)phenyl]-6-({[2-(4-morpholinyl)-1,3-thiazol-4-yl]-methyl}sulfanyl)-3,5-pyridindicarbonitril

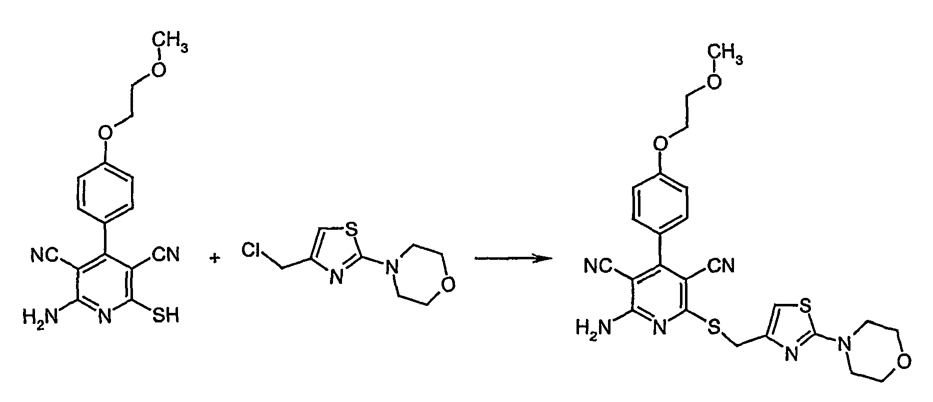

2 g (6.13 mmol) 2-Amino-4-[4-(2-methoxyethoxy)phenyl]-6-sulfanyl-3,5-pyridin-dicarbonitril und 2.68 g (12.26 mmol) 4-[4-(Chlormethyl)-1,3-thiazol-2-yl]morpholin werden in trockenem DMF (50 ml) gelöst und mit 1.83 ml (12.26 mmol) DBU versetzt. Nach 3 Stunden Rühren bei RT wird das Solvens am Rotationsverdampfer abgezogen und der Rückstand durch präparative HPLC gereinigt (Säule: Kromasil 100 C18 250 x 20 mm, 10 µm; Acetonitril-Wasser-Gradient: 3 Minuten 10 % Acetonitril, dann innerhalb 30 Minuten auf 80 % Acetonitril; Flussrate: 25 ml/min). Man erhält 1.70 g (55 % d.Th.) Produkt.
MS (ESIpos): m/z = 509 (M+H)⁺
¹H-NMR (300 MHz, DMSO-d₆): δ = 3.3 (m, 7H); 3.7 (m, 6H); 4.2 (tr, 2H); 4.4 (s, 2H); 6.95 (s, 1H); 7.15 (d, 2H); 7.45 (d, 2H); 8.0 (s, breit, 2H).

Analog zu Beispiel 13 wurden die in Tabelle 3 aufgeführten Beispiele hergestellt. Die als Einsatzstoff verwendeten Chlormethylthiazole sind entweder kommerziell erhältlich oder können analog zu Stufe 1 in Beispiel 13 hergestellt werden.

**Tabelle 3**

| **Beispiel-Nr.** | **Struktur** | **gesuchte Molmasse** | **gefundenes [M+H]⁺** |
|---|---|---|---|
| **14** | 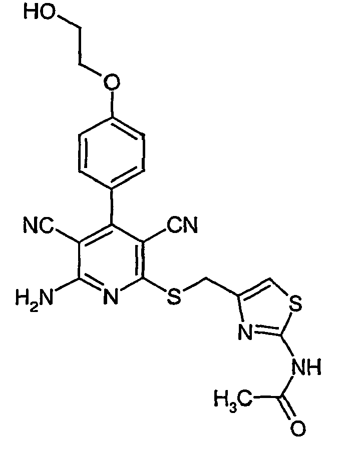 | 467 | 468 |
| **15** | 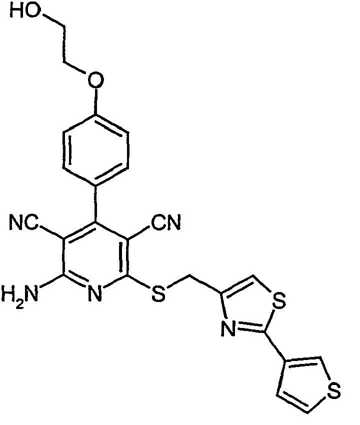 | 492 | 493 |
| **16** | 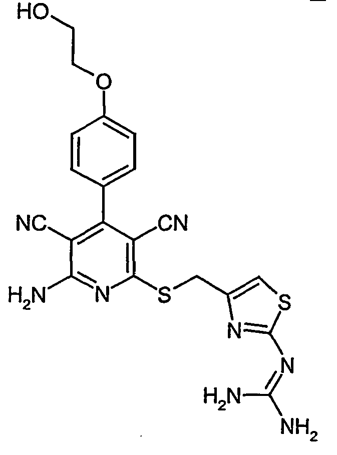 | 467 | 468 |
| **17** | 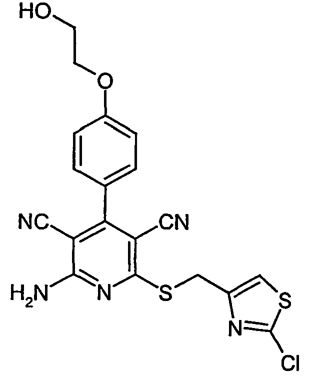 | 444 | 445 |
| **18** | 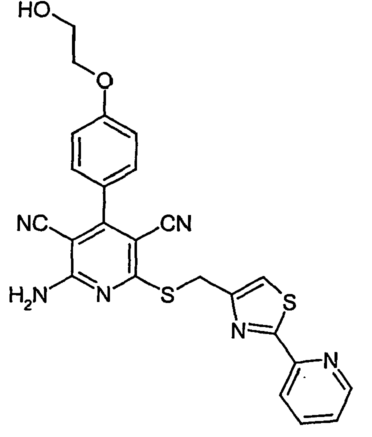 | 487 | 488 |
| **19** | 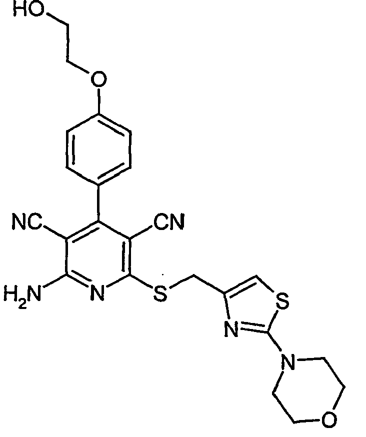 | 495 | 496 |
| **20** | 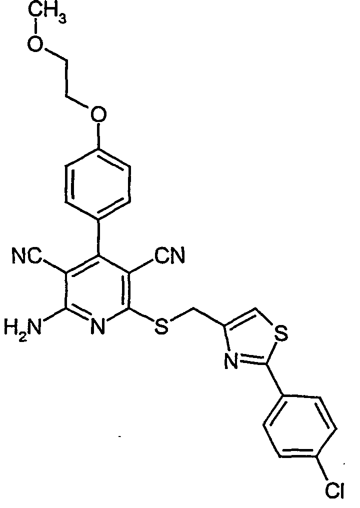 | 534 | 535 |
| **21** | 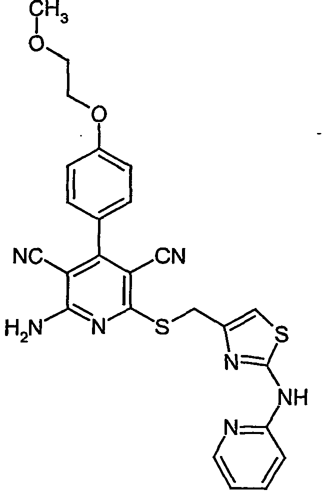 | 516 | 517 |
| **22** | 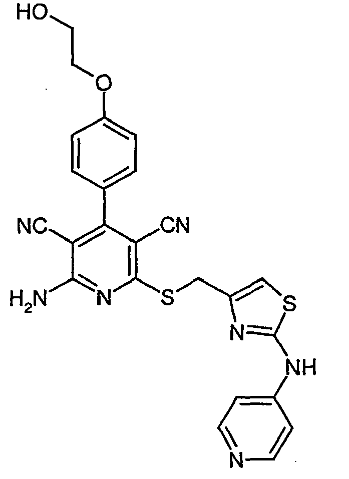 | 502 | 503 |
| **23** | 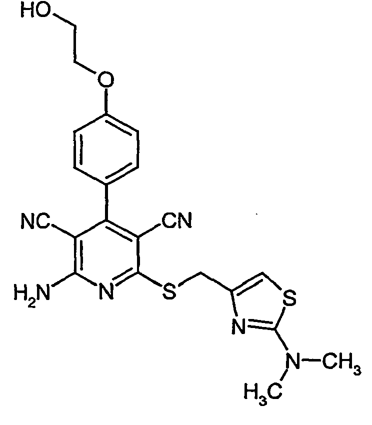 | 453 | 454 |
| **24** | 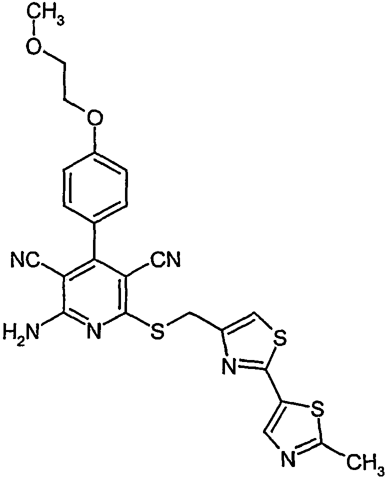 | 521 | 522 |
| **25** | 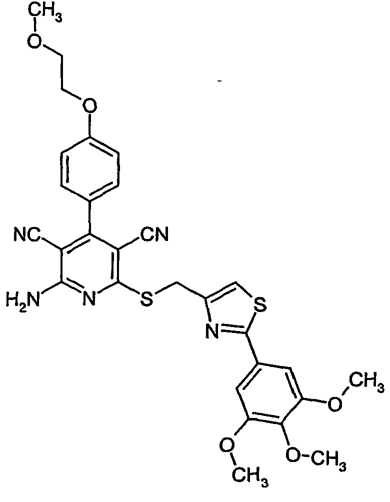 | 590 | 591 |
| **26** | 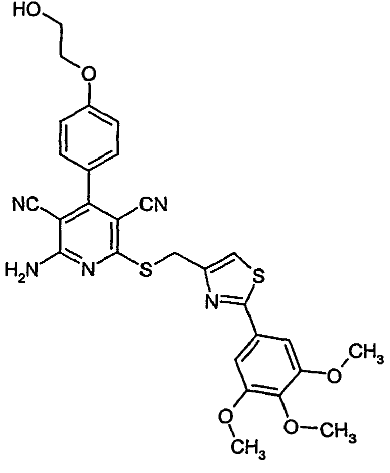 | 576 | 577 |
| **27** | 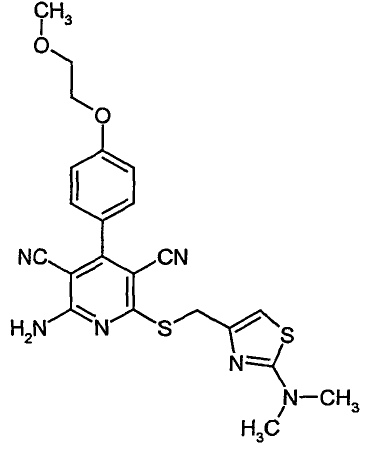 | 467 | 468 |

## Patentansprüche

1. Verbindungen der Formel (I) 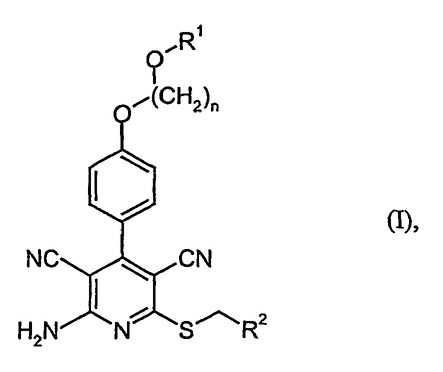 worin
n eine Zahl 2, 3 oder 4 bedeutet,
R¹ Wasserstoff oder (C₁-C₄)-Alkyl bedeutet
und
R² Pyridyl oder Thiazolyl bedeutet, das seinerseits durch (C₁-C₄)-Alkyl, Halogen, Amino, Dimethylamino, Acetylamino, Guanidino, Pyridyl-amino, Thienyl, Furyl, Imidazolyl, Pyridyl, Morpholinyl, Thiomorpholinyl, Piperidinyl, Piperazinyl, N-(C₁-C₄)-Alkylpiperazinyl, Pyrrolidinyl, Oxazolyl, Isoxazolyl, Pyrimidinyl, Pyrazinyl, gegebenenfalls durch (C₁-C₄)-Alkyl substituiertes Thiazolyl oder gegebenenfalls bis zu dreifach durch Halogen, (C₁-C₄)-Alkyl oder (C₁-C₄)-Alkoxy substituiertes Phenyl substituiert sein kann,
und ihre Salze, Hydrate, Hydrate der Salze und Solvate.

2. Verbindungen der Formel (I) nach Anspruch 1,
worin
n die Zahl 2 bedeutet,
R¹ Wasserstoff, Methyl oder Ethyl bedeutet
und
R² Pyridyl oder Thiazolyl bedeutet, das seinerseits durch Methyl, Ethyl, Fluor, Chlor, Amino, Dimethylamino, Acetylamino, Guanidino, 2-Pyridylamino, 4-Pyridylamino, Thienyl, Pyridyl, Morpholinyl, Piperidinyl, gegebenenfalls durch Methyl substituiertes Thiazolyl oder gegebenenfalls bis zu dreifach durch Chlor oder Methoxy substituiertes Phenyl substituiert sein kann,
und ihre Salze, Hydrate, Hydrate der Salze und Solvate.

3. Verbindungen der Formel (I) nach Anspruch 1,
worin
n die Zahl 2 bedeutet,
R¹ Wasserstoff oder Methyl bedeutet
und
R² Pyridyl oder Thiazolyl bedeutet, das seinerseits durch Methyl, Chlor, Amino, Dimethylamino, Acetylamino, Guanidino, 2-Pyridylamino, 4-Pyridylamino, Thienyl, Pyridyl, Morpholinyl, 2-Methyl-thiazol-5-yl, Phenyl, 4-Chlorphenyl oder 3,4,5-Trimethoxyphenyl substituiert sein kann,
und ihre Salze, Hydrate, Hydrate der Salze und Solvate.

4. Verbindung nach einem der Ansprüche 1 bis 3 mit der folgenden Struktur 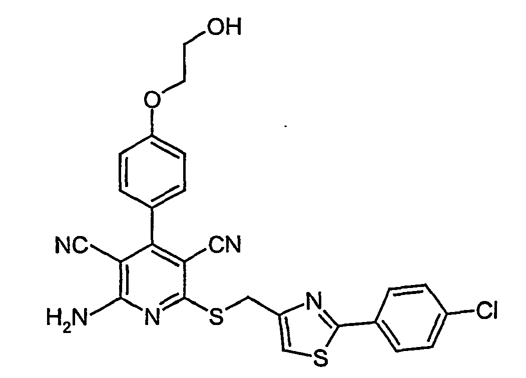 und ihre Salze, Hydrate, Hydrate der Salze und Solvate.

5. Verfahren zur Herstellung der Verbindungen der Formel (I), wie in Anspruch 1 definiert, **dadurch gekennzeichnet, dass** man Verbindungen der Formel (II) 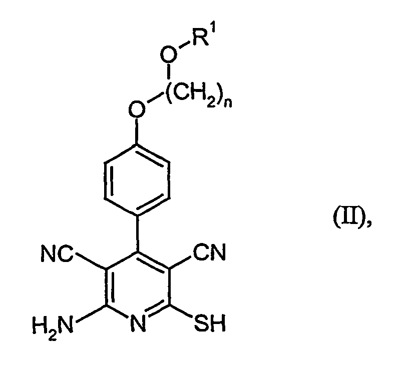 worin
n und R¹ die in Anspruch 1 angegebene Bedeutung haben,
mit Verbindungen der Formel (III)
R²-CH₂-X (III),
worin
R² die in Anspruch 1 angegebene Bedeutung hat und X für eine Abgangsgruppe steht,
umsetzt.

6. Verbindungen der Formel (I), wie in Anspruch 1 definiert, zur Prophylaxe und/oder Behandlung von Erkrankungen.

7. Arzneimittel, enthaltend mindestens eine Verbindung der Formel (I), wie in Anspruch 1 definiert, und mindestens einen Hilfsstoff.

8. Arzneimittel, enthaltend mindestens eine Verbindung der Formel (I), wie in Anspruch 1 definiert, und mindestens einen weiteren Wirkstoff.

9. Verwendung von Verbindungen der Formel (I), wie in Anspruch 1 definiert, zur Herstellung von Arzneimitteln zur Prophylaxe und/oder Behandlung von Erkrankungen des Herzkreislaufsystems.

10. Verwendung von Verbindungen der Formel (I), wie in Anspruch 1 definiert, zur Herstellung von Arzneimitteln zur Prophylaxe und/oder Behandlung von Erkrankungen des Urogenitalbereichs und Krebs.

11. Verwendung von Verbindungen der Formel (I), wie in Anspruch 1 definiert, zur Herstellung von Arzneimitteln zur Prophylaxe und/oder Behandlung von inflammatorischen und neuroinflammatorischen Erkrankungen, neurodegenerativen Erkrankungen und Schmerzzuständen.

## Claims

1. Compounds of the formula (I) 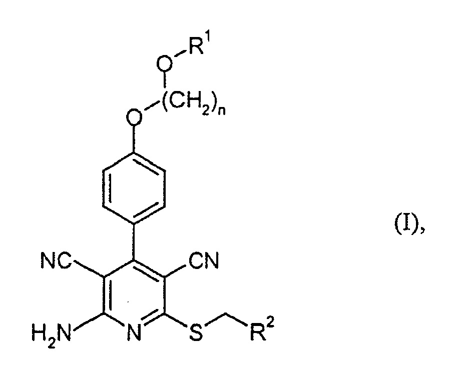 in which
n represents a number 2, 3 or 4,
R¹ represents hydrogen or (C₁-C₄) -alkyl
and
R² represents pyridyl or thiazolyl which for its part may be substituted by (C₁-C₄) -alkyl, halogen, amino, dimethylamino, acetylamino, guanidino, pyridylamino, thienyl, furyl, imidazolyl, pyridyl, morpholinyl, thiomorpholinyl, piperidinyl, piperazinyl, N-(C₁-C₄)-alkylpiperazinyl, pyrrolidinyl, oxazolyl, isoxazolyl, pyrimidinyl, pyrazinyl, optionally (C₁-C₄)-alkyl-substituted thiazolyl or phenyl which is optionally substituted up to three times by halogen, (C₁-C₄)-alkyl or (C₁-C₄) -alkoxy,
and their salts, hydrates, hydrates of the salts and solvates.

2. Compounds of the formula (I) according to Claim 1,
in which
n represents the number 2,
R¹ represents hydrogen, methyl or ethyl,
and
R² represents pyridyl or thiazolyl which for its part may be substituted by methyl, ethyl, fluorine, chlorine, amino, dimethylamino, acetylamino, guanidino, 2-pyridylamino, 4-pyridylamino, thienyl, pyridyl, morpholinyl, piperidinyl, optionally methyl-substituted thiazolyl or phenyl which is optionally substituted up to three times by chlorine or methoxy,
and their salts, hydrates, hydrates of the salts and solvates.

3. Compounds of the formula (I) according to Claim 1,
in which
n represents the number 2,
R¹ represents hydrogen or methyl
and
R² represents pyridyl or thiazolyl which for its part may be substituted by methyl, chlorine, amino, dimethylamino, acetylamino, guanidino, 2-pyridylamino, 4-pyridylamino, thienyl, pyridyl, morpholinyl, 2-methylthiazol-5-yl, phenyl, 4-chlorophenyl or 3,4,5-trimethoxyphenyl,
and their salts, hydrates, hydrates of the salts and solvates.

4. Compound according to any of Claims 1 to 3 having the following structure 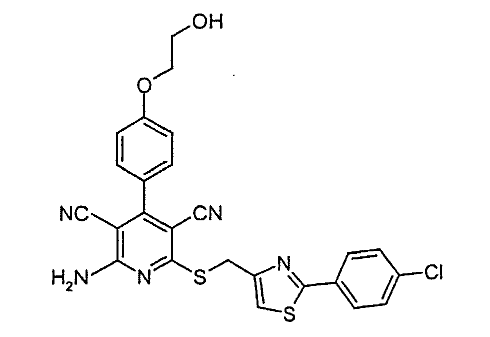 and its salts, hydrates, hydrates of the salts and solvates.

5. Process for preparing compounds of the formula (I) as defined in Claim 1, **characterized in that** compounds of the formula (II) 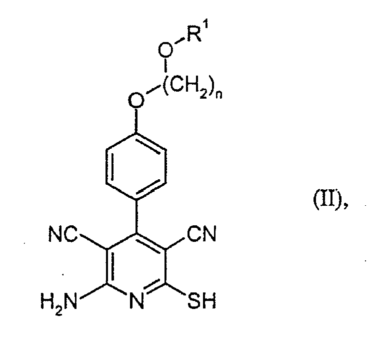 in which
n and R¹ are as defined in Claim 1,
are reacted with compounds of the formula (III)
R²-CH₂-X (III),
in which
R² is as defined in Claim 1 and X represents a leaving group.

6. Compounds of the formula (I) as defined in Claim 1 for the prophylaxis and/or treatment of disorders.

7. Medicaments, comprising at least one compound of the formula (I) as defined in Claim 1 and at least one auxiliary.

8. Medicaments, comprising at least one compound of the formula (I) as defined in Claim 1 and at least one further active compound.

9. Use of compounds of the formula (I) as defined in Claim 1 for preparing medicaments for the prophylaxis and/or treatment of disorders of the cardiovascular system.

10. Use of compounds of the formula (I) as defined in Claim 1 for preparing medicaments for the prophylaxis and/or treatment of disorders of the urogenital region and cancer.

11. Use of compounds of the formula (I) as defined in Claim 1 for preparing medicaments for the prophylaxis and/or treatment of inflammatory and neuroinflammatory disorders, neurodegenerative disorders and pain.

## Revendications

1. Composés de formule (I) 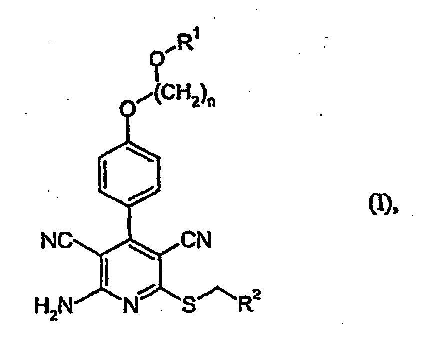 dans laquelle
n représente le chiffre 2, 3 ou 4,
R¹ désigne l'hydrogène ou un alkyle en C₁-C₄
et
R² est un pyridyle ou un thiazolyle, qui peut lui-même être substitué par un alkyle en C₁-C₄, un halogène, un amino, un diméthylamino, un acétylamino, un guanidino, un pyridylamino, un thiényle, un furyle, un imidazolyle, un pyridyle, un morpholinyle, un thiomorpholinyle, un pipéridinyle, un pipérazinyle, un N-alkyl(en C₁-C₄)-pipérazinyle, un pyrrolidinyle, un oxazolyle, un isoxazolyle, un pyrimidinyle, un pyrazinyle, un thiazolyle substitué le cas échéant par un alkyle en C₁-C₄, ou un phényle substitué le cas échéant jusqu'à trois fois par un halogène, un alkyle en C₁-C₄ ou un alcoxy en C₁-C₄, ainsi que les sels, hydrates, hydrates de sels et solvates de ceux-ci.

2. Composés de formule (I) selon la revendication 1,
dans laquelle
n représente le chiffre 2,
R¹ désigne l'hydrogène, un méthyle ou un éthyle
et
R² est un pyridyle ou un thiazolyle, qui peut lui-même être substitué par un méthyle, un éthyle, un fluoro, un chloro, un amino, un diméthylamino, un acétylamino, un guanidino, un 2-pyridylamino, un 4-pyridylamino, un thiényle, un pyridyle, un morpholinyle, un pipéridinyle, un thiazolyle substitué le cas échéant par un méthyle ou un phényle substitué le cas échéant jusqu'à trois fois par un chloro ou un méthoxy,
ainsi que les sels, hydrates, hydrates de sels et solvates de ceux-ci.

3. Composés de formule (I) selon la revendication 1,
dans laquelle
n représente le chiffre 2,
R¹ désigne l'hydrogène ou un méthyle
et
R² est un pyridyle ou un thiazolyle, qui peut lui-même être substitué par un méthyle, un chloro, un amino, un diméthylamino, un acétylamino, un guanidino, un 2-pyridylamino, un 4-pyridylamino, un thiényle, un pyridyle, un morpholinyle, un 2-méthyl-thiazol-5-yle, un phényle, un 4-chlorophényle ou un 3,4,5-triméthoxyphényle,
ainsi que les sels, hydrates, hydrates de sels et solvates de ceux-ci.

4. Composé selon une des revendications 1 à 3 présentant la structure suivante 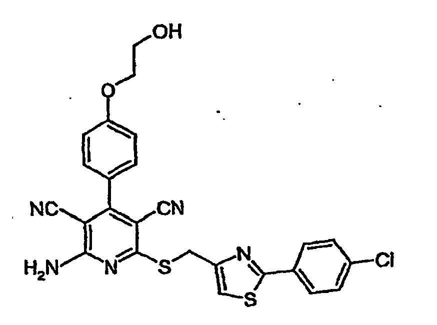 ainsi que les sels, hydrates, hydrates de sels et solvates de ceux-ci.

5. Procédé de préparation des composés de formule (I), tels qu'ils sont définis dans la revendication 1, **caractérisé en ce que** l'on fait réagir des composés de formule (II) 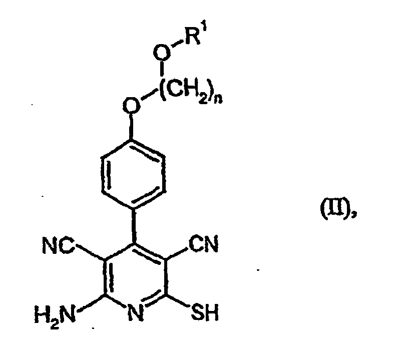 dans laquelle n et R¹ possèdent la signification mentionnée dans la revendication 1
avec des composés de formule (III)
R²-CH₂-X (III),
dans laquelle
R² possède la signification mentionnée dans la revendication 1 et X représente un groupe de départ.

6. Composés de formule (I), tels qu'ils sont définis dans la revendication 1, pour la prophylaxie et/ou le traitement de maladies.

7. Médicaments contenant au moins un composé de formule (I), tel qu'il est défini dans la revendication 1 et au moins un excipient.

8. Médicaments contenant au moins un composé de formule (I), tel qu'il est défini dans la revendication 1 et au moins un autre excipient.

9. Utilisation de composés de formule (I), tels qu'ils sont définis dans la revendication 1, pour la préparation de médicaments pour la prophylaxie et/ou le traitement de maladies cardiovasculaires.

10. Utilisation de composés de formule (I), tels qu'ils sont définis dans la revendication 1, pour la préparation de médicaments pour la prophylaxie et/ou le traitement de maladies urogénitales et du cancer.

11. Utilisation de composés de formule (I), tels qu'ils sont définis dans la revendication 1, pour la préparation de médicaments pour la prophylaxie et/ou le traitement de maladies inflammatoires et neuroinflammatoires, d'affections neurodégénératives et d'états douloureux.
